# EUROPEAN PATENT APPLICATION

(11) **EP 4 368 634 A1**
(43) Date of publication of application: **15.05.2024**
(21) Application number: 22837925.1
(22) Date of filing: 05.07.2022
(51) Int. Cl.: C07K 14/33, C12N 9/64, C07K 1/20

(54) **METHOD FOR PURIFYING CLOSTRIDIUM BOTULINUM TOXIN COMPLEX PROTEIN**

(30) Priority: 05.07.2021 KR 20210087988
(71) Applicant: Pharma Research Bio Co., Ltd., Gangneung-si, Gangwon-do 25451 (KR)
(72) Inventor: BAEK, Seung Gul, Seongnam-si, Gyeonggi-do 13486 (KR); KIM, Hyung Gun, Seongnam-si, Gyeonggi-do 13486 (KR); KIM, Jeong Han, Seongnam-si, Gyeonggi-do 13486 (KR)
(74) Representative: Cabinet Beau de Loménie
(86) International application number: PCT/KR2022/009641
(87) International publication number: WO 2023/282573

(57) **Abstract**

The present invention relates to a method of purifying a *Clostridium botulinum* toxin complex protein.

## Description

### [Technical Field]

The present invention relates to a method of purifying a *Clostridium botulinum* toxin complex protein.

### [Background Art]

Botulinum toxin is a type of neurotoxic protein produced by bacteria such as *Clostridium botulinum,* and binds irreversibly to the presynaptic nerve terminals, which results in the inhibition of acetylcholine release in synapses, thereby blocking muscle contraction and exhibiting the secondary effect of muscle relaxation. Due to these functions, botulinum toxin has been used for therapeutic and cosmetic purposes since approval by the US FDA in 1989 (KR 10-2010-0107475 A, KR 10-2008-0049152 A, etc.).

Botulinum toxin is used for therapeutic purposes in neuromuscular diseases such as strabismus, torticollis, or blepharospasm, and for cosmetic purposes in removing wrinkles or frown lines, and treating square jaw, hyperhidrosis, or migraine as an injectable formulation. As side effects, cases such as dysphagia, voice change, dry mouth, blurred vision, etc. have been reported, but there have been no direct reports of deaths caused by botulinum toxin yet. Therefore, botulinum toxin is evaluated as a very safe drug when used appropriately.

Meanwhile, botulinum toxin naturally consists of a complex bound to several non-toxic proteins.

### [Disclosure]

### [Technical Problem]

The present inventors intend to provide an improved purification method of isolating a stable and biologically active botulinum toxin complex protein.

### [Technical Solution]

An object of the present invention is to provide a method of purifying a *Clostridium botulinum* toxin complex protein.

### [Advantageous Effects]

By using a purification method of the present invention, a highly pure botulinum toxin complex protein may be obtained economically and efficiently.

### [Brief Description of the Drawing]

FIG. 1 shows the results of SDS-PAGE, i.e., sodium dodecyl sulfate polyacrylamide gel electrophoresis of a purified botulinum toxin complex protein (900 kD) (NTNH: nontoxic nonhemagglutinin, HC: heavy chain of neurotoxic protein, LC: light chain of neurotoxic protein, HA33: hemagglutinin 33, HA17: hemagglutinin 17, HA50: hemagglutinin 50, HA20: hemagglutinin 20).
FIG. 2 shows the results of HPLC, i.e., size exclusion chromatography (SEC-HPLC) of the purified 900 kD-botulinum toxin complex protein.

### [Detailed Description of Preferred Embodiments]

The present invention will be described in detail as follows. Meanwhile, each description and embodiment disclosed in this disclosure may also be applied to other descriptions and embodiments. That is, all combinations of various elements disclosed in this disclosure fall within the scope of the present invention. Further, the scope of the present invention is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present invention.

An aspect of the present invention provides a method of purifying a *Clostridium botulinum* toxin complex protein.

Specifically, the purification method may include the steps of:
(a) loading, on a primary hydrophobic interaction column, a solution including the *Clostridium botulinum* toxin complex protein isolated from a culture to capture the toxin and to pass impurities;
(b) isolating the toxin captured in the step (a) to obtain an eluent including the toxin;
(c) loading, on a secondary hydrophobic interaction column, the eluent obtained in the step (b) to capture the toxin and to pass impurities;
(d) isolating the toxin captured in the step (c) to obtain an eluent including the toxin; and
(e) performing size exclusion chromatography using the eluent obtained in the step (d).

In the present invention, there is no time interval between the steps such as "(a), (b), (c), (d)...", or the steps may be carried out simultaneously, or the steps may be performed at any time intervals of seconds, minutes, hours, etc.

As used herein, the term "*Clostridium botulinum* toxin" is, also referred to as "botulinum toxin", a type of proteins derived from *Clostridium botulinum,* and refers to a protein that binds irreversibly to the presynaptic nerve terminals, which results in the inhibition of acetylcholine release in synapses, thereby blocking muscle contraction and exhibiting the secondary effect of muscle relaxation.

Botulinum toxin protein has a molecular weight of about 150 kD, and is divided into 7 types from A to G according to serological characteristics. Botulinum toxin type A is the most lethal natural substance known to humans, and in addition to serotype A, other six serotypes of botulinum toxins which are generally immunologically distinct have been identified, i.e., botulinum toxin serotypes B, C, D, E, F, and G. Different serotypes may be identified by neutralization with type-specific antibodies. The different serotypes of botulinum toxin vary in the severity of the paralysis they evoke and in the animal species that they affect.

The molecular weight of the botulinum toxin protein molecule, for all seven of the known botulinum toxin serotypes, is about 150 kD. Meanwhile, the botulinum toxins are released by *Clostridial bacterium* as complexes including the 150 kD botulinum toxin protein molecule along with associated non-toxin proteins. Thus, the botulinum toxin type A complex may be produced by *Clostridial bacterium* as 900 kD, 500 kD and 300 kD forms. Botulinum toxin types B and C may be produced as 500 kD complexes, and botulinum toxin type D may be produced as 300 kD and 500 kD complexes. Botulinum toxin types E and F may be produced as 300 kD complexes. These complexes (i.e., molecular weight greater than about 150 kD) are believed to include a non-toxin hemagglutinin protein and a non-toxin and non-toxic non-hemagglutinin protein.

The botulinum toxin protein includes a high-molecular-weight complex form containing a pure neurotoxic component of about 150 kD and non-toxin proteins. Thus, the complexed form may include the botulinum neurotoxin protein and one or more non-toxic hemagglutinin proteins, and/or one or more non-toxic non-hemagglutinin protein. Specifically, the botulinum toxin complex protein may be a complex of botulinum neurotoxin (BoNT), non-toxic non-hemagglutinin (NTNH), and hemagglutinin (HA) proteins. The molecular weight of the complex may be higher than about 150 kD. For example, the complexed form of the botulinum toxin type A may have a molecular weight of about 900 kD, about 500 kD, or about 300 kD. A method of purifying the *Clostridium botulinum* toxin complex of the present invention may purify the various complexes.

In one embodiment, the botulinum toxin of the present invention may be type A toxin.

In any one embodiment of the above-described embodiments, the botulinum toxin of the present invention may be in the form of a complex of being bound with a non-toxic protein. Specifically, the botulinum toxin complex protein of the present invention may be a complex of botulinum toxin (BoNT), non-toxic non-hemagglutinin (NTNH), and hemagglutinin (HA) proteins. More specifically, the botulinum toxin complex protein of the present invention may be a complex of BoNT, NTNH, hemagglutinin 70 (HA70), hemagglutinin 33 (HA33), and hemagglutinin 17 (HA17). More specifically, the HA70 may be divided into hemagglutinin 20 (HA20) and hemagglutinin 50 (HA50), or the BoNT may be divided into about 50 kD light chain (LC) and about 100 kD heavy chain (HC), but is not limited thereto.

In any one embodiment of the above-described embodiments, the botulinum toxin complex protein of the present invention may have a molecular weight of more than 150 kD. Specifically, it may have a molecular weight of about 250 kD to 1400 kD, more specifically, a molecular weight of 280 kD to 1300 kD, 300 kD to 1200 kD, 700 kD to 1100 kD, 800 kD to 1000 kD, or about 900 kD, but is not limited thereto.

As used herein, the term "about" includes the exact number following the term as well as approximately the number or ranges near to the number. Considering the context in which the number is presented, whether a number is near to or approximately a specifically recited number may be determined. For example, the term "about" may refer to a range of -10% to +10% of a numeric value. For another example, the term "about" may refer to a range of -5% to +5% of a given numerical value. However, it is not limited thereto.

In any one embodiment of the above-described embodiments, the *Clostridium botulinum* toxin complex protein of the present invention may be obtained from a culture of *Clostridium botulinum.* Specifically, the protein may be isolated from the culture of *Clostridium botulinum.*

Therefore, the purification method of the present invention may further include the step of culturing *Clostridium botulinum,* prior to the chromatography step for purification, but is not limited thereto.

In the culturing step, the culturing of the *Clostridium botulinum* strain may be performed according to an appropriate medium and culture conditions known in the art. Specifically, the *Clostridium botulinum* strain may be cultured in a common medium containing appropriate carbon sources, nitrogen sources, phosphorus sources, inorganic compounds, etc. under anaerobic conditions while controlling temperature, pH, etc.

For example, the culturing may be performed under anaerobic conditions at about 25°C to 40°C, specifically, at 27°C to 40°C, but is not limited thereto. The culturing period may continue until a desired production amount of the toxin protein is obtained, specifically, for about 12 hours to 150 hours, but is not limited thereto.

In any one embodiment of the above-described embodiments, the solution including the *Clostridium botulinum* toxin complex protein may be subjected to precipitation treatment prior to the primary hydrophobic chromatography step.

In one embodiment, the precipitation may be acid precipitation, but is not limited thereto. For example, the acid precipitation may include adding an acid to pH 3.0 to pH 4.0, specifically pH 3.3 to 3.5, or more specifically pH 3.4 to 3.5. In the acid precipitation, an acidic solution known in the art, for example, sulfuric acid or hydrochloric acid, etc. may be used, but is not limited thereto.

In any one embodiment of the above-described embodiments, the solution including the *Clostridium botulinum* toxin complex protein of the present invention may be subjected to filtration treatment prior to the primary hydrophobic chromatography step. Specifically, the solution may be subjected to filtration treatment after the acid precipitation.

The filtration step may be performed by a known process such as microfiltration, ultrafiltration, precision filtration, depth filtration, etc., and impurities may be removed in the filtration step.

In one embodiment, a device used in the filtration treatment may include a filter having a pore size of about 0.1 µm to about 0.3 µm, specifically about 0.2 µm, but is not limited thereto.

In the purification method of the present invention, the steps of (a) loading, on a primary hydrophobic interaction column, the solution including the *Clostridium botulinum* toxin complex protein to capture the toxin and to pass impurities; and (b) isolating the toxin captured in the step (a) to obtain an eluent including the toxin may be referred to as the step of performing primary hydrophobic interaction chromatography using the solution including the *Clostridium botulinum* toxin complex protein, but is not limited thereto.

As used herein, the "chromatography" refers to any process of separating components of a mixture by passing the mixture through a medium such that the components of the mixture pass through the medium at different rates.

The chromatography of the present invention includes column chromatography, planar chromatography, thin layer chromatography, gas chromatography, liquid chromatography, fast protein liquid chromatography (FPLC), and high performance liquid chromatography (HPLC). In each step of the purification process of the present invention, an exemplary type of chromatography process or device is disclosed, which may be applied to all types of chromatography described above.

As used herein, the "hydrophobic chromatography" or "hydrophobic interaction chromatography" is a method of separating molecules based on the relative strength of hydrophobic interaction with a nonpolar stationary phase. The hydrophobic chromatography uses the principle that the higher the salt concentration, the stronger the interaction between a non-polar stationary phase and a substance to be separated, and the lower the ionic strength or salt concentration of a buffer, the weaker the interaction. Therefore, when a descending gradient of the salt concentration is used, less hydrophobic substances may be eluted first, and more hydrophobic substances may be eluted later.

In one embodiment, in the step (a) of the purification method of the present invention, a column having ligands such as ether, isopropyl, butyl, octyl, phenyl, etc. may be used as the hydrophobic interaction column. For example, in the step (a) of the purification method of the present invention, the hydrophobic interaction column may be a butyl sepharose or phenyl sepharose column. Specifically, the hydrophobic interaction column may be a phenyl sepharose column. More specifically, the hydrophobic interaction column may be selected from the group consisting of butyl sepharose high performance (butyl sepharose HP), butyl sepharose fast flow, phenyl sepharose high performance (HP), and phenyl sepharose fast flow columns, but is not limited thereto, and any column may be used without limitation, as long as it belongs to hydrophobic interaction columns.

In any one embodiment of the above-described embodiments, the step of equilibrating the column using a buffer solution may be further included prior to the step (a) of the purification method of the present invention.

As used herein, the equilibration may refer to the step of stabilizing the column with a buffer solution in order to prevent a protein from aggregating or losing activity due to changes in the environment before injecting, into the column, the protein to be purified.

In the equilibration step prior to the step (a), conditions such as a flow rate of flowing the buffer solution, temperature, time, conductivity, etc. may be appropriately adjusted.

For example, a phosphate buffer solution, a citrate buffer solution, or an acetate buffer solution may be used as the buffer solution. For example, the column buffer solution may be a phosphate buffer solution, for example, sodium phosphate.

For example, the flow rate may be about 1.0 ml/min to about 20.0 ml/min. For example, the conductivity may be about 170 mS/cm to about 220 mS/cm, but is not limited thereto.

In any one embodiment of the above-described embodiments, the step (a) of the purification method of the present invention may be performed under pH condition of pH 4 to pH 8, but is not limited thereto.

In any one embodiment of the above-described embodiments, the step (a) of the purification method of the present invention may be performed under conductivity condition of about 170 mS/cm to about 220 mS/cm, but is not limited thereto.

In the step (a) and/or the step (b), the flow rate of flowing the solution, temperature, time, etc. may be appropriately adjusted.

For example, a phosphate buffer solution, a citrate buffer solution, or an acetate buffer solution may be used as the column buffer solution. For example, the column buffer solution may be a phosphate buffer solution, for example, sodium phosphate. For example, the concentration of the column buffer solution may be adjusted to about 5 mM to about 100 mM, e.g., 25 mM to 75 mM, or 40 mM to 60 mM. For example, the flow rate of the mobile phase may be about 1.0 ml/min to about 20.0 ml/min, but is not limited thereto.

In any one embodiment of the above-described embodiments, in the step (b), an appropriate elution solvent may be used in the step of obtaining an eluent including the toxin complex protein.

The step (b) may use a concentration gradient. For example, the toxin complex protein may be eluted using a stepwise salt gradient or continuous salt gradient.

The step (b) may include reducing ionic strength or increasing pH. In the elution step, the hydrophobic moiety of the protein which is adsorbed to the stationary phase may be desorbed to the mobile phase by, for example, starting a reverse salt gradient to reduce the salt concentration. Specifically, in the elution step, a descending concentration gradient of a buffering agent may be used. For example, a buffering agent with a concentration gradient ranging from about 5.0 M to about 0.0 M, about 4.0 M to about 0.0 M, about 3.5 M to about 0.0 M, or about 3.0 M to about 0.0 M may be used. The buffering agent may be, for example, sodium sulfate (Na₂SO₄), sodium chloride (NaCl), potassium chloride (KCl), ammonium acetate (NH₄OAc), etc., and may be specifically sodium chloride (NaCl), but is not limited thereto. A buffering agent appropriately selected from the range known in the art may be used.

In any one embodiment of the above-described embodiments, the eluent obtained in the step (b) of the purification method of the present invention may be subjected to precipitation treatment prior to performing the step (c). For example, the precipitation may be acid precipitation.

The acid precipitation may include adding an acid. Specifically, the acid precipitation may be adding ammonium sulfate to the eluent. More specifically, the acid precipitation may be adding ammonium sulfate to the eluent so that the final saturation is about 30% to about 50%. For example, the concentration of ammonium sulfate at the saturation level may be about 19 g/100 ml to 32 g/100 ml, but is not limited thereto.

The precipitated eluent may undergo additional purification processes, for example, filtration and/or centrifugation, etc. The obtained precipitate may be re-dissolved for additional purification processes. For example, the precipitate may be dissolved using a solution with a pH of 5.0 to 7.0. For example, a sodium phosphate buffer solution with a concentration of about 40 mM to about 60 mM may be used, but is not limited thereto.

In the purification method of the present invention, the step (c) of loading, on a secondary hydrophobic interaction column, the eluent obtained in the step (b) to capture the toxin and to pass impurities; and the step (d) of isolating the toxin captured in the step (c) to obtain an eluent including the toxin may be referred to as the step of performing secondary hydrophobic interaction chromatography using the eluent obtained by performing the primary chromatography. The hydrophobic interaction chromatography is as described above.

In one embodiment, in the step (c) of the purification method of the present invention, a column having ligands such as ether, isopropyl, butyl, octyl, phenyl, etc. may be used as the secondary hydrophobic interaction column. For example, in the step (c) of the purification method of the present invention, the hydrophobic interaction column may be a butyl sepharose or phenyl sepharose column. Specifically, the hydrophobic interaction column may be a phenyl sepharose column. More specifically, the hydrophobic interaction column may be selected from the group consisting of butyl sepharose high performance (butyl sepharose HP), butyl sepharose fast flow, phenyl sepharose high performance (HP), and phenyl sepharose fast flow columns, but is not limited thereto, and any column may be used without limitation, as long as it belongs to hydrophobic interaction columns.

In any one embodiment of the above-described embodiments, the step of equilibrating the column may be further included prior to the step (c) of the purification method of the present invention.

The buffer solution may be, for example, a phosphate buffer solution, a citrate buffer solution, or an acetate buffer solution. For example, the column buffer solution may be a phosphate buffer solution, for example, sodium phosphate.

The flow rate may be, for example, about 1.0 ml/min to about 20.0 ml/min. The conductivity may be, for example, about 130 mS/cm to about 170 mS/cm, but is not limited thereto.

In any one embodiment of the above-described embodiments, the step (c) of the purification method of the present invention may be performed under condition of pH 4 to pH 8, but is not limited thereto.

In any one embodiment of the above-described embodiments, the step (c) of the purification method of the present invention may be performed under conductivity condition of 130 mS/cm to 170 mS/cm, but is not limited thereto.

In the step (c) and/or the step (d), the flow rate of flowing the solution, temperature, time, etc. may be appropriately adjusted.

For example, a phosphate buffer solution, a citrate buffer solution, or an acetate buffer solution may be used as the column buffer solution. For example, the column buffer solution may be a phosphate buffer solution, for example, sodium phosphate. For example, the concentration of the column buffer solution may be adjusted to about 5 mM to about 100 mM, e.g., 25 mM to 75 mM, or 40 mM to 60 mM. For example, the flow rate of the mobile phase may be about 1.0 ml/min to about 20.0 ml/min, but is not limited thereto.

In any one embodiment of the above-described embodiments, an appropriate elution solvent may be used in the step of obtaining an eluent including the toxin complex protein in the step (d).

The step (d) may use a concentration gradient. For example, the toxin complex protein may be eluted using a stepwise salt gradient or continuous salt gradient.

The step (d) may include reducing ionic strength or increasing pH. In the elution step, the hydrophobic moiety of the protein which is adsorbed to the stationary phase may be desorbed to the mobile phase by, for example, starting a reverse salt gradient to reduce the salt concentration. Specifically, in the elution step, a descending concentration gradient of a buffering agent may be used. For example, a buffering agent with a concentration gradient ranging from about 2.5 M to about 0.0 M, about 2.3 M to about 0.0 M, about 2.1 M to about 0.0 M, about 2.0 M to about 0.0 M may be used. The buffering agent may be, for example, sodium sulfate (Na₂SO₄), sodium chloride (NaCl), potassium chloride (KCl), ammonium acetate (NH₄OAc), etc., and may be specifically sodium chloride (NaCl), but is not limited thereto. A buffering agent appropriately selected from the range known in the art may be used.

In any one embodiment of the above-described embodiments, the eluent obtained in the step (d) of the purification method of the present invention may be subjected to precipitation treatment prior to performing the step (e). For example, the precipitation may be acid precipitation.

The acid precipitation may include adding an acid. Specifically, the acid precipitation may be adding ammonium sulfate to the eluent. More specifically, the acid precipitation may be adding ammonium sulfate to the eluent so that the final saturation is about 70% to about 90%. For example, the concentration of ammonium sulfate at the saturation level may be about 47 g/100 ml to 67 g/100 ml, but is not limited thereto.

The precipitated eluent may undergo additional purification processes, for example, filtration and/or centrifugation, etc. The obtained precipitate may be re-dissolved for additional purification processes. For example, the precipitate may be dissolved using a solution with a pH of 5.0 to 7.0. For example, a sodium phosphate buffer solution with a concentration of about 10 mM to about 30 mM may be used, but is not limited thereto.

In the purification method of the present invention, components may be separated by molecular size through the step (e) of size exclusion chromatography (SEC). For example, a botulinum toxin complex protein with a specific size may be isolated from the step (e). As used herein, the "size exclusion chromatography (SEC)" refers to separation of a mixture based on the speed (permeability) at which various sizes of solutes pass through a porous matrix. The size exclusion chromatography uses the principle that when a sample to be analyzed is passed through a column packed with a porous stationary phase, such as gel, matrix, or beads, large molecules that are not able to pass through the pores of the column are excluded from the pores and quickly come out of the column through the surrounding empty space, whereas small molecules move relatively slowly to come out through the column while passing through the pores of the column.

In one embodiment, the column which may be used in the size exclusion chromatography of the present invention may be a superdex, sephacryl, superose, sephadex, sepharose, polyacrylamide, or silica-based column, specifically, a superose column, and more specifically, superose-6, but is not limited, as long as it is able to isolate the desired botulinum toxin complex protein.

For example, as the column buffer solution of the size exclusion chromatography, a phosphate buffer solution, a citrate buffer solution, or an acetate buffer solution may be used. For example, the column buffer solution may be a phosphate buffer solution, for example, sodium phosphate. For example, the concentration of the column buffer solution may be adjusted to about 5 mM to about 100 mM, e.g., about 5 mM to about 50 mM, or about 10 mM to about 30 mM. For example, the buffer solution may be a buffer solution with a pH of 5 to 7. For example, the flow rate may be about 0.001 ml/min to about 10.0 ml/min, or about 0.05 ml/min to about 0.5 ml/min, but is not limited thereto.

In any one embodiment of the above-described embodiments, 250 kD to 1400 kD of botulinum toxin complex proteins may be isolated through the size exclusion chromatography step of the present invention, but is not limited thereto.

The botulinum toxin complex protein isolated according to the purification method of the present invention may have a high purity. Specifically, the botulinum toxin complex protein may have a purity of about 90% or more, about 95% or more, about 96% or more, about 97% or more, about 97.5% or more, or about 98% or more, but is not limited thereto.

### [Mode for Carrying Out the Invention]

Hereinafter, the present invention will be described in more detail with reference to Examples and Experimental Examples. However, these Examples and Experimental Examples are only for illustrating the present invention, and the scope of the present invention is not intended to be limited by these Examples and Experimental Example.

### Example 1. Culture of botulinum bacteria and Isolation of toxin

*Clostridium botulinum* stored at -80°C was thawed and added to a seed culture medium, and cultured at 37°C under anaerobic conditions for 24 hours to increase the number of bacteria. The culture medium in which the number of bacteria was increased was added to a main culture medium and further cultured at 35°C under anaerobic conditions for 92 hours to 100 hours, and then virus inactivation was performed through acid precipitation to adjust pH to 3.4. When precipitation was completed, the botulinum toxin was eluted with a buffer solution, and then filtered through a 0.2 µm sterile filter without contact with the outside.

### Example 2. Purification of botulinum toxin

### 2-1. Primary hydrophobic interaction chromatography

The filtered solution containing the botulinum type A toxin of Example 1 was loaded onto a column packed with a phenyl sepharose hydrophobic interaction chromatography resin. Before the loading, equilibration/washing was performed by applying an equilibration/elution buffer solution (50 mM sodium phosphate, 3 M sodium chloride, pH 6.0) at a flow rate of 5 ml/min and a conductivity of 188 mS/cm.

The filtered solution containing the botulinum type A toxin of Example 1 was loaded onto a phenyl sepharose column, and then botulinum toxin was eluted from the column using a descending salt gradient (3.0 M - 0.0 M sodium chloride gradient) of a buffering agent of 50 mM sodium phosphate with a pH of 6.0 under conditions of a flow rate of 5 ml/min and a conductivity of 188 mS/cm.

### 2-2. Ammonium sulfate precipitation

Precipitation was performed by adding ammonium sulfate to the eluent collected by the method of Example 2-1 at a final saturation of 40% (24.3 g/100 ml), and then centrifugation was performed to obtain a precipitate. In this process, the toxin protein was also in contact with the inner surface of the sterile container, and there was no contact with the outside. The precipitate was dissolved in a 50 mM sodium phosphate buffer solution (pH 6.0), and then secondary hydrophobic interaction chromatography was performed.

### 2-3. Secondary hydrophobic interaction chromatography

The re-dissolved solution of Example 2-2 was centrifuged to separate the supernatant, and the separated supernatant was injected to fast protein liquid chromatography (FPLC) connected to a phenyl sepharose hydrophobic interaction chromatography column.

Before injection of the supernatant, the column packed with a phenyl sepharose resin was equilibrated by applying an equilibrium buffer solution (50 mM sodium phosphate, 2M sodium chloride, pH 6.0) thereto, and the buffer solution (50 mM sodium phosphate, 2 M sodium chloride buffer solution, pH 6.0) containing the toxin was applied to the column at a flow rate of 5 ml/min and a conductivity of 147 mS/cm. After equilibration, the injection step was performed, and the flow through (FT) fluid was collected in a sterile container while the toxin complex bound to the hydrophobic interaction column material, followed by the washing step. After washing, the toxin was eluted from the column using a descending salt gradient (2.0 M - 0.0 M sodium chloride gradient) of a buffering agent of 50 mM sodium phosphate with a pH of 6.0.

### 2-4. Secondary ammonium sulfate precipitation and concentration

Precipitation was performed by adding ammonium sulfate to the eluent collected by the method of Example 2-3 at a final saturation of 80% (56.1 g/100 ml), and then centrifugation was performed to obtain a precipitate. In this process, the toxin protein was also in contact with the inner surface of the sterile container, and there was no contact with the outside. The precipitate was dissolved in 1 ml of a 20 mM sodium phosphate buffer solution (pH 6.0), and then size exclusion chromatography was performed.

### 2-5. Size exclusion chromatography

The re-dissolved solution of Example 2-4 was centrifuged to separate the supernatant, and the separated supernatant was loaded onto fast protein liquid chromatography (FPLC) connected to a superose-6 size exclusion chromatography column. Before loading, the column was equilibrated by applying an equilibration/elution buffer solution (20 mM sodium phosphate, pH 6.0) at a flow rate of 0.2 ml/min. When equilibration was completed, 100 mL of a 20 mM sodium phosphate buffer solution with a pH of 6.0 was applied, and then fractions of the eluted peak were sequentially obtained to separate a sample containing a protein of approximately 900 kD in size.

### Example 3. Purity analysis of purified toxin

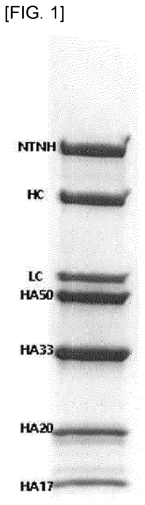

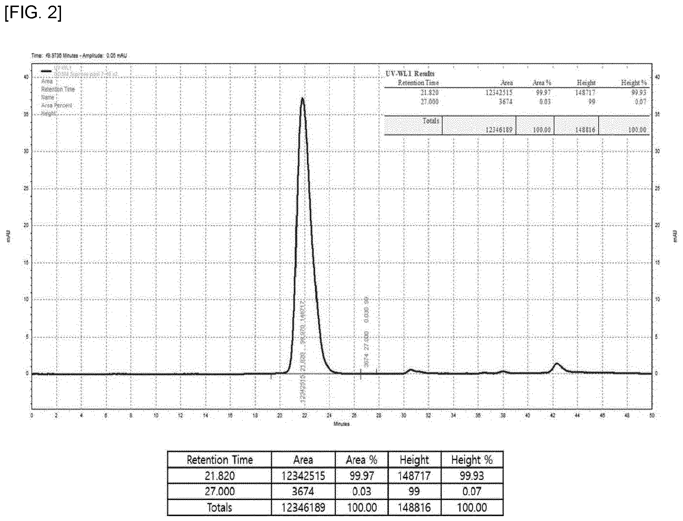

In order to purify the complex neurotoxin of high purity, the sample which was obtained through the purification process of two cycles of hydrophobic interaction chromatography and size exclusion chromatography performed in Example 2 was subjected to a purity analysis through sodium dodecyl sulfate polyacrylamide gel electrophoresis (SDS-PAGE, FIG. 1) and size exclusion chromatography (SEC-HPLC, FIG. 2). Electrophoresis was performed using a 4-20% polyacrylamide gel, followed by staining with Coomassie blue. As a mobile phase of SEC-HPLC, a 50 mM sodium phosphate solution containing 250 mM NaCl with a pH of 6.0 was used, and an HPLC column was connected, and 20 µg of botulinum type A toxin protein obtained in Example 2 was loaded and applied at a rate of 0.4 mL/mL for 60 minutes.

As a result, no impurity protein bands were observed except for the botulinum complex neurotoxin component (FIG. 1). Further, it was confirmed that the purity of botulinum toxin was 99.97% (FIG. 2). Therefore, when the above purification method is used, the botulinum toxin complex proteins (about 900 kD), such as botulinum toxin type A, etc., may be obtained in high purity without impurities.

These results indicate that the botulinum complex protein may be isolated and purified in high purity using the method and system disclosed in this specification.

Based on the above description, it will be understood by those skilled in the art that the present invention may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of purifying a *Clostridium botulinum* toxin complex protein, the method comprising the steps of:
(a) loading, on a primary hydrophobic interaction column, a solution including the *Clostridium botulinum* toxin complex protein isolated from a culture to capture the toxin and to pass impurities;
(b) isolating the toxin captured in the step (a) to obtain an eluent including the toxin;
(c) loading, on a secondary hydrophobic interaction column, the eluent obtained in the step (b) to capture the toxin and to pass impurities;
(d) isolating the toxin captured in the step (c) to obtain an eluent including the toxin; and
(e) performing size exclusion chromatography using the eluent obtained in the step (d).

2. The method of claim 1, comprising the step of culturing a *Clostridium botulinum* strain prior to the step (a).

3. The method of claim 2, further comprising the step of performing acid precipitation of the culture of the strain.

4. The method of claim 3, wherein the acid precipitation includes adding an acid to pH 3.0 to pH 4.0.

5. The method of claim 3, further comprising the step of filtering the acid-precipitated solution.

6. The method of claim 1, wherein the step (a) is performed under conditions of pH 4 to pH 8; and conductivity of 170 mS/cm to 220 mS/cm.

7. The method of claim 1, wherein the column buffer solution of the step (a) is a phosphate buffer solution.

8. The method of claim 1, wherein a concentration gradient is used in the step (b).

9. The method of claim 1, further comprising the step of performing acid precipitation of the eluent obtained in the step (b), between the step (b) and the step (c).

10. The method of claim 9, wherein the acid precipitation step includes adding ammonium sulfate to the eluent at a final saturation of 30% to 50%.

11. The method of claim 1, wherein the step (c) is performed under conditions of pH 4 to pH 8; and conductivity of 130 mS/cm to 170 mS/cm.

12. The method of claim 1, wherein the column buffer solution of the step (c) is a phosphate buffer solution.

13. The method of claim 1, wherein a concentration gradient is used in the step (d).

14. The method of claim 1, further comprising the step of performing acid precipitation of the eluent obtained in the step (d), between the step (d) and the step (e).

15. The method of claim 14, wherein the acid precipitation step includes adding ammonium sulfate to the eluent at a final saturation of 70% to 90%.

16. The method of claim 1, wherein the hydrophobic interaction column is selected from the group consisting of butyl sepharose and phenyl sepharose columns.

17. The method of claim 1, wherein the step (e) includes isolating and obtaining a protein having a molecular weight of 250 kD to 1400 kD.

18. The method of claim 1, wherein a superdex, sephacryl, superose, sephadex, sepharose, polyacrylamide, or silica-based column is used in the size exclusion chromatography of the step (e).

19. The method of claim 1, wherein the size exclusion chromatography of the step (e) is performed under condition of pH 5 to pH 7.

20. The method of claim 1, wherein the column buffer solution of the step (e) is a phosphate buffer solution.

21. The method of claim 1, wherein the *Clostridium botulinum* toxin complex protein purified by the method has a purity of 98% or more.

22. The method of any one of claims 1 to 21, wherein the *Clostridium botulinum* toxin complex protein is a complex of botulinum toxin (BoNT), nontoxic nonhemagglutinin (NTNH), and hemagglutinin (HA) proteins.
